# EUROPEAN PATENT SPECIFICATION

(11) **EP 2 085 053 B1**
(45) Date of publication and mention of the grant of the patent: **04.05.2011**
(21) Application number: 09001503.3
(22) Date of filing: 04.02.2009
(51) Int. Cl.: A61F 2/16

(54) **Intraocular lens injection instrument**
Einspritzinstrument für eine Intraokularlinse
Instrument d'injection de lentille intraoculaire

(30) Priority: 04.02.2008 JP 2008024462; 04.02.2008 JP 2008024463
(43) Date of publication of application: 05.08.2009
(73) Proprietor: Nidek Co., Ltd., Gamagori-shi, Aichi 443-0038 (JP)
(72) Inventor: Nagasaka, Shinji, Gamagori-shi Aichi 443-0038 (JP); Sunada, Chikara, Gamagori-shi Aichi 443-0038 (JP); Oda, Haruo, Gamagori-shi Aichi 443-0038 (JP)
(74) Representative: Prüfer & Partner GbR European Patent Attorneys

(56) References cited:
- EP-A- 1 360 946
- EP-A- 1 424 048
- EP-A- 1 502 559
- WO-A-2009/010751

## Description

### Technical Field

The present invention relates to an intraocular lens injection instrument for injecting an intraocular lens in an eye.

### Background Art

As one of operative treatments for cataract, heretofore, there has generally been used a method in which a lens is removed from a patient's eye and then a foldable, soft intraocular lens (IOL) is injected in place of the lens. For injection of such foldable IOL, an IOL injection instrument called an injector is used. With this injector, the IOL is injected in a folded state into the eye through an incision. The incision therefore has only to be formed in advance with a minimum diameter in the eye. This injector is configured to advance the IOL set therein by a push rod called a plunger while the IOL is folded to a smaller size, and push out the IOL through a tip end of the injector (for example, see Patent Literature 1). Such injector has a passage having an almost constant inner diameter and a passage having a gradually decreasing inner diameter so that the IOL passes through the passages while changing from an unfolded state or a slightly folded state to a folded (rolled) state to a smaller size.

### Citation List

### Patent Literature

Patent Literature 1: EP1502559A1 Patent Literature 2: EP 1 360 946 A1 which forms the basis of the preamble of claim 1.

### Technical Problem

In the conventional injector as disclosed in Patent Literature 1, the IOL is pushed to pass through the passage having an almost constant inner diameter and then the passage having a gradually decreasing inner diameter. Accordingly, operating pressure (push-out load) tends to change during an IOL push-out operation. Thus, the conventional injector has a disadvantage that, even when the IOL is lodged or stuck somewhere in the passages and accordingly the operating pressure decreases, such an abnormal state could not easily be perceived. Furthermore, the operating pressure (push-out load) of the plunger is high while the IOL is in a rolled, smaller state during the push-out operation. However, as the IOL starts to be moved out from the tip end of the injector, a frictional force between the IOL and an inner wall of the injector becomes lower, and thus the operating pressure is likely to suddenly change, making the plunger hard to treat.

The present invention has an object to overcome the above problems and to provide an intraocular lens injection instrument capable of appropriately adjusting changes in operating pressure during a push-out operation of an intraocular lens.
Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

### Solution to Problem

In order to solve the foregoing problems, the present invention provides an intraocular lens injection instrument comprising: a lens holding part (10) comprising: a setting part (12) in which an intraocular lens (IOL) is placeable; and an injection part (11) having a taper tubular shape for rolling up the IOL placed in the setting part; a main body (20) having a tubular shape and holding the lens holding part in a front end; and a push-out member (30) including a push rod (31) provided to be movable in an axial direction thereof inside the main body and the lens holding part to push the IOL out of the injection part, characterized in that a member provided in the main body or the lens holding part is internally formed with an interfering part (16) that will contact with the push-out member to generate a frictional force to adjust operating pressure of the push-out member during forward movement of the push-out member, and the push-out member is applied with treatment or a shape for discontinuously changing a contact state with the interfering part in the axial direction to change the frictional force with respect to the interfering part according to a position of the IOL with respect to the lens holding part.

Further developments of the present invention are given in the dependent claims.

### Advantageous Effects of Invention

According to the present invention, it is possible to reduce changes in operating pressure during a push-out operation of an intraocular lens.

### Brief Description of Drawings

In the drawings,
Figs. 1A and 1B are external views of an intraocular lens insertion instrument in a preferred embodiment;
Fig. 2 is a structural view of an intraocular lens (IOL);
Figs. 3A and 3B are external views of a cartridge in the embodiment;
Figs. 4A and 4B are views showing a folding manner of the IOL by deformation of the cartridge;
Fig. 5 is a perspective external view of a cylindrical unit;
Fig. 6 is a sectional side view of the IOL insertion instrument;
Fig. 7 is a schematic sectional view showing a state in which a head portion contacts with an optic part;
Fig. 8 is a schematic sectional view showing a state in which the optic part has reached an injection part;
Fig. 9 is a schematic sectional view showing a state in which the optic part is rolled to a smaller diameter by an internal shape of the injection part;
Fig. 10 is a schematic sectional view showing a state in which part of the optic part is moved out from a tip end of the injection part; and
Fig. 11 is a schematic sectional view showing a state in which the entire optic part is moved out from the injection part.

### Description of Embodiments

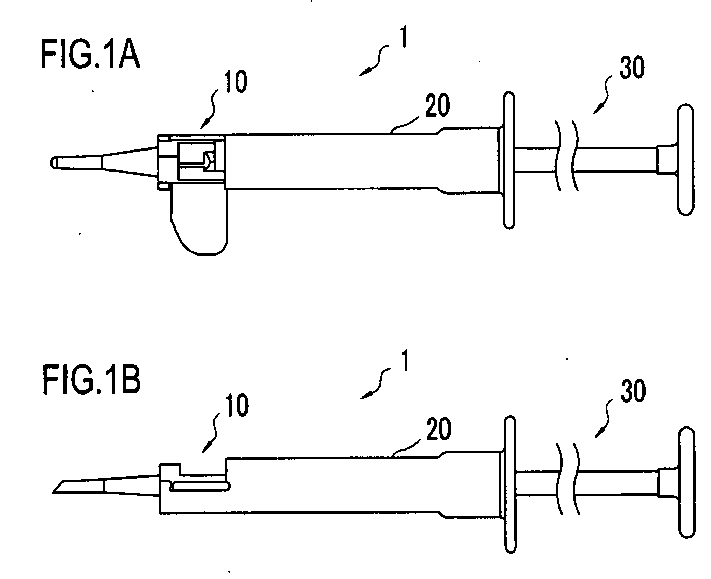

A detailed description of a preferred embodiment of the present invention will now be given referring to the accompanying drawings. Figs. 1A and 1B are schematic external views of an IOL injection instrument 1 used in this embodiment; specifically, Fig. 1A is a top view of the IOL injection instrument 1 and Fig. 1B is a side view of the same.

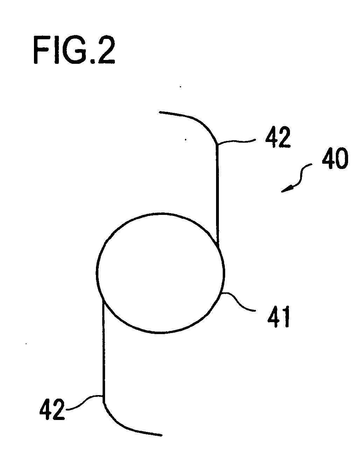

The IOL injection instrument 1 includes, in the order of insertion into an eye, a lens holding part (hereinafter, a "cartridge") 10 for holding an intraocular lens (IOL) 40 (see Fig. 2), the cartridge 10 including an injection part for inserting the IOL 40 into the eye through an incision made in the eye and a setting part in which the IOL 40 is to be set; a cylindrical unit (a main body, a hand piece) 20 having a tubular configuration including a tubular part and a front end in which the cartridge 10 is mountable (placeable); and a push-out member (a plunger) 30 placed to be movable through the insides of the cartridge 10 and the cylindrical unit 20 and configured to push out the IOL 40 from a tip end of the cartridge 10 mounted in the cylindrical unit 20.

Fig. 2 is a view showing a configuration of the IOL 40. This IOL 40 includes an optic part 41 having predetermined refractive power and a pair of support parts 42, called an open loop, for fixedly supporting the optic part 41 in the eye. The optic part 41 of the IOL 40 used in this embodiment is made of a material commonly used for a typical foldable soft intraocular lens, for example, a monomeric material such as hydroxyethyl methacrylate (HEMA) and a composite material of acrylic ester and methacrylate ester. The support parts 42 are also made of a material commonly used for a typical IOL support part such as polymethylmetacrylate (PMMA). The IOL 40 used in this embodiment is a three-piece IOL produced in such a way that the optic part 41 and the thin-loop-shaped (C-shaped or J-shaped) support parts 42 are separately made of the aforementioned materials and then assembled together.

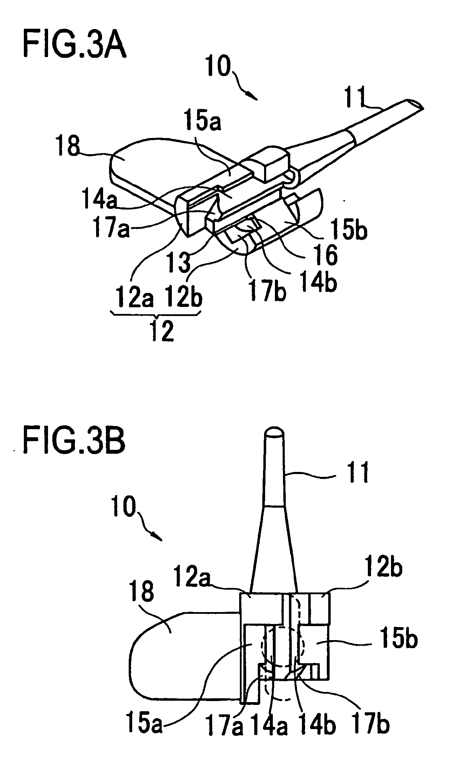

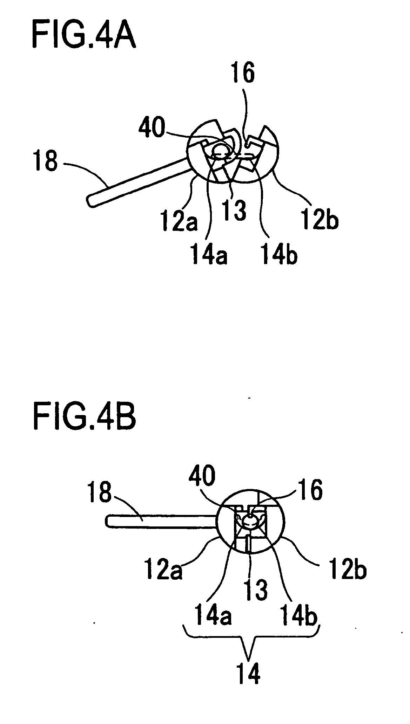

Figs. 3A, 3B, 4A, and 4B are views showing a configuration of the cartridge 10. As shown in the figures, the cartridge 10 integrally includes an injection part (injection tube) 11 having a tapered shape whose diameter becomes smaller (narrower) toward the tip end and a setting part 12 in which the IOL 40 is to be set. It is to be noted that the cartridge 10 is entirely made of synthetic resin as a disposable type which is to be thrown away after one use. Accordingly, the cartridge 10 is preferably made of resin or the like by molding.

The setting part 12 is constituted of two half-split elements 12a and 12b. As shown in Fig. 3A, the half-split elements 12a and 12b are connected to each other at respective lower edges by a hinge 13 so as to be opened and closed. The half-split elements 12a and 12b include setting stages 14a and 14b respectively for mounting thereon the IOL 40. The shapes (wall shapes) of the setting stages 14a and 14b are curved in a direction to fold the IOL 40. The injection part 11 provided at a rear end of the setting part 12 is formed in a hollow tubular shape through which the folded IOL 40 can be pushed out from a tip end of the injection part 11.

When the half-split elements 12a and 12b are closed into contact with each other, the shapes of walls (setting surfaces) of the setting stages 14a and 14b are transformed to substantially conform in cross section to the shape (semi-circle) of an opening of the injection part 11 on a rear end side (see Figs. 4A and 4B). The outer shape of the setting part 12 with the half-split elements 12a and 12b being closed is substantially equal to the shape of an inner wall of the cylindrical unit 20 mentioned later. As shown in Fig. 3B, the setting stage 14a (14b) is sized to allow the support part 42 which is a rear one (a lower one in Fig. 3B) (i.e., the one opposite from the injection part 11) to slightly extend out of the setting stage 14a when the optic part 41 (indicated by a broken line) of the IOL 40 is set on the setting stage 14a (14b).

In the IOL injection instrument 1 in this embodiment, the rear support part 42 of the IOL 40 is positioned on a left side with respect to an advancing direction of the IOL 40 when the IOL 40 is set in the cartridge 10. Furthermore, the total length of the half-split element 12a is determined to be longer than the total length of the half-split element 12b so that the half-split element 12a is longer on a rear end side of the cartridge 10. Such a difference in total length between the half-split elements 12a and 12b can provide the space for receiving the support part 42 positioned on the rear side when the cartridge 10 is mounted so that the rear support part 42 is off the central axis of a push rod (a push axis) 31 mentioned later.

Covers 15a and 15b are provided as upper portions of the half-split elements 12a and 12b respectively to cover over the setting stages 14a and 14b when the half-split elements 12a and 12b are closed. The cover 15b is provided at an end with a protrusion 16 protruding from above and toward the setting stages 14a and 14b and extending by a predetermined length in an axial direction. The protrusion 16 serves to guide a bending (folding) direction of the IOL 40 set in the cartridge 10 to a direction along the inner walls (the setting surfaces) of the setting stages 14a and 14b. The length of the protrusion 16 in the axial length is longer than the width of the optic part 41 so that the protrusion 16 reaches at least a rear side of the IOL 40 when set on the setting stage 14. Thus, the protrusion 16 is formed at an upper portion of an inner wall of a passage (a movement passage) through which a push rod 31 moves. Furthermore, the protrusion 16 will contact with part of the push rod 31 which is moved forward to push out the IOL 40. Therefore, the protrusion 16 also serves as an interfering part or interfering means that will generate a frictional force and increase operating pressure (push-out load) of the push-out member 30 to a level required for pushing out the IOL 40.

Slant portions 17a and 17b are provided for facilitating insertion of the IOL 40 from the rear end side of the cartridge 10 onto the setting stages 14a and 14b. A flat-plate-like grip 18 is provided on the side of the half-split element 12a. The grip 18 will be grasped by a user to hold the cartridge 10.

As shown in Fig. 4A, the IOL 40 is set on the setting stages 14a and 14b of the cartridge 10 while the setting part 12 is opened (the half-split elements 12a and 12b are separated). Successively, the cartridge 10 is mounted in the cylindrical unit 20 by closing the half-split elements 12a and 12b as shown in Fig. 4B, thereby applying stress on the set IOL 40 to fold it. Accordingly, the cartridge 10 has an internal structure that can fold the IOL 40. An inner passage in the injection part 11 has a shape becoming narrower toward the tip end. The IOL 40 folded in the setting part 12 is further reduced (folded or rolled) to a smaller diameter in passing through the inner passage in the injection part 11 and is moved out from the tip end of the injection part 11.

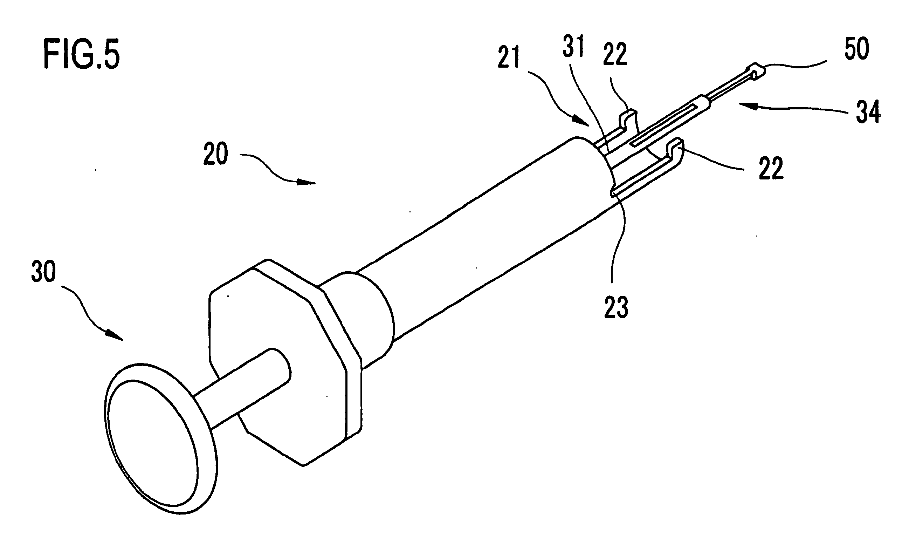

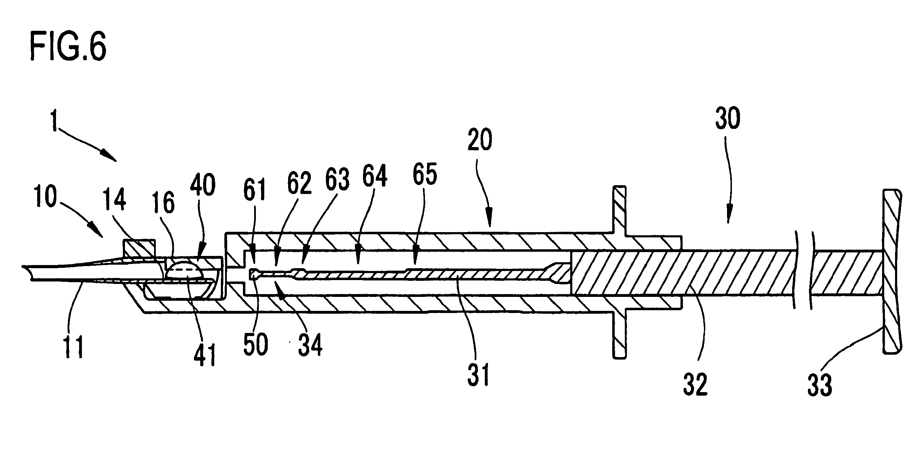

Fig. 5 is a schematic perspective external view of the cylindrical unit 20. The details of an internal structure of this cylindrical unit 20 are explained referring to Fig. 6. As shown in the figures, the cylindrical unit 20 is provided, at a front end, with a mounting part 21 in which the cartridge 10 is to be removably mounted. The mounting part 21 is of a nearly half split shape of the front end portion of the cylindrical unit 20. The mounting part 21 is formed with protrusions 22 at a front end and recesses 23 at a rear end, each of which are symmetric with respect to the push rod 31 extending along the central axis of the cylindrical unit 20. The protrusions 22 are positioned slightly above the central axis of the cylindrical unit 20 and have a distance therebetween slightly narrower (shorter) than the inner diameter of the cylindrical unit 20. Each protrusion 22 has a shape for a snap-in configuration serving as a guide for causing the opened half-split elements 12a and 12b to move in a closing direction when the cartridge 10 is mounted in the mounting part 21 and limiting the width of the half-split elements 12a and 12b, and also for reliably holding the mounted cartridge 10 against coming-off from the cylindrical 20. Right and left edges of the mounting part 21 also serve as a guide as with the protrusions 22 to cause the half-split elements 12a and 12b to close. The push rod 31 is formed at its distal end with a head portion 50 (the details thereof will be mentioned later) which will contact with the optic part 41 of the IOL 40.

Next, the internal structure of the IOL injection instrument 1 assembled from the cartridge 10 and the cylindrical unit 20 is explained below. Fig. 6 is a schematic sectional view of the IOL injection instrument 1. In this figure, the cartridge 10 is mounted in the mounting part 21 and thus the IOL 40 is placed in a folded state. For convenience of explanation, the support parts 42 are not illustrated.

As shown in Fig. 6, the inside of the cylindrical unit 20 is hollow, in which the push-out unit 30 is inserted so as to be movable forward and backward in an axial direction in a passage continuous from the cylindrical unit 20 to the tip end of the cartridge 10 (the injection part 11). The push-out member 30 includes the push rod 31, a base part 32, and a press part 33. The push rod 31 has the head portion 50 at the distal, the base part 32 is integrally provided at one end of the press part 33 and is held in contact with the inner wall of the cylindrical unit 20, and the press part 33 is to be pressed by a surgeon (a user) to push out the IOL 40.

The push rod 31 is made of an axial rod having a smaller diameter than the base part 32 and is attached to a front end of the base part 32. When the press part 33 is pressed in an axial direction to move the base part 32 forward, the IOL 40 is pushed forward out of the cartridge 10 mounted in the front end of the cylindrical unit 20. The push rod 31 (the push-out member 30) serves to move the IOL 40 from the injection part 11 to the outside. Accordingly, the push rod 31 is formed with such a size (diameter) as to be passable through the inner passage in the injection part 11 and the cartridge 10 and be smaller than the base part 32. A distal end portion of the push rod 31 includes a narrow portion 34 having a smaller diameter and a predetermined axial length to prevent the rear support part 42 of the IOL 40 set in the cartridge 10 from tangling (becoming caught) between the push rod 31 and the passage and hence becoming damaged when the IOL 40 is to be pushed out. Furthermore, the head portion 50 that will contact with the IOL 40 is formed at a front end (an extreme forward end) of the narrow portion 34. The diameter of the head portion 50 is designed to be equal to or smaller than the diameter of the push rod 31. The narrow portion 34 has a length needed to avoid interference with the rear support part 42 (i.e., tangling of the support part 42) during push-out of the IOL 40.

The push rod 31 is designed so that an upper portion (an upper surface) partially has a height enough to contact with the protrusion 16 in a push-out step. Herein, as shown in Fig. 6, the push rod 31 includes, in the order of positions from the head portion 50 side to a rear side, a contact portion 61, a recess 62, a contact portion 63, a recess 64, and a contact portion 65 which are axially formed in an outer surface of the push rod 31. The recesses 62 and 64 are inwardly recessed from the outermost periphery of the push rod 31 to avoid contact with the protrusion 16 during forward movement of the push rod 31. The contact portions 61, 63, and 65 are formed to have a height equal to the head portion 50. To be more concrete, the diameter (thickness) of portions of the push rod 31 corresponding to the head portion 50 and the contact portions 61, 63, and 65 is determined to be slightly larger (by about 0.01 mm to about 0.2 mm) than a distance (an interval) between a setting surface of the setting stage 14 and the protrusion 16. The diameter of the push rod 31 needed to contact with the setting surface of the setting stage 14 and the protrusion 16 has only to be determined so as to provide a fit condition producing operating pressure almost equal to operating pressure needed to push out the IOL 40 folded (rolled) to a smaller size in the vicinity of the tip end of the injection part 11. In the figure, the contact portions 61, 63, and 65 and the recesses 62 and 64, which are schematically illustrated, are preferably continuous with each other by smooth or obtuse ridges. Thus, the push rod 31 can smoothly contact with the protrusion 16 in the push-out step.

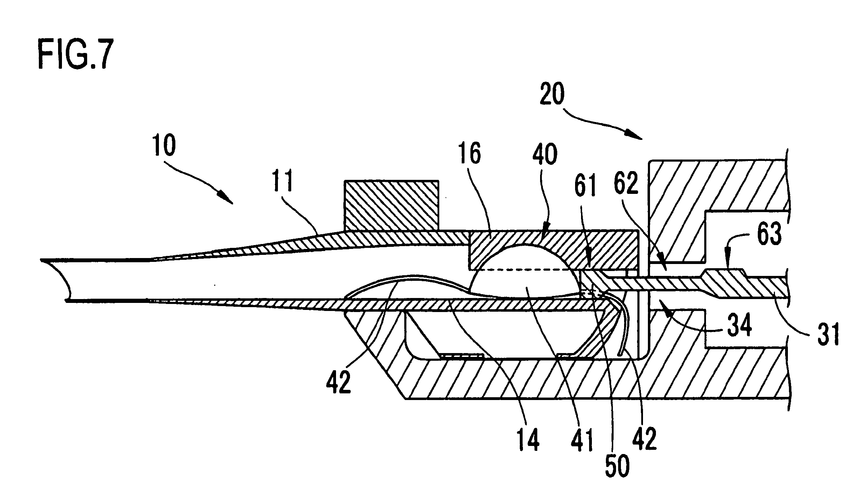

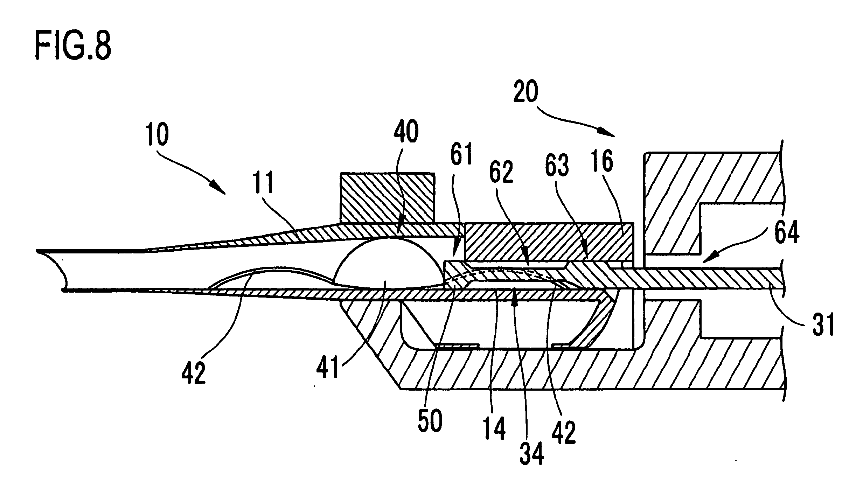

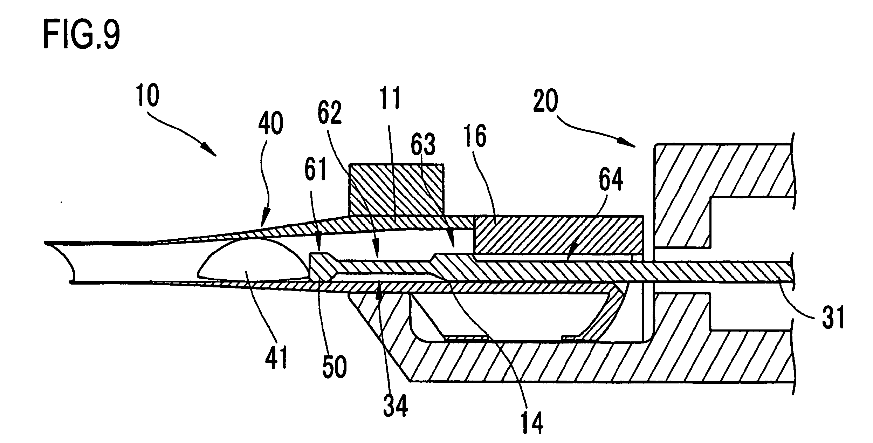

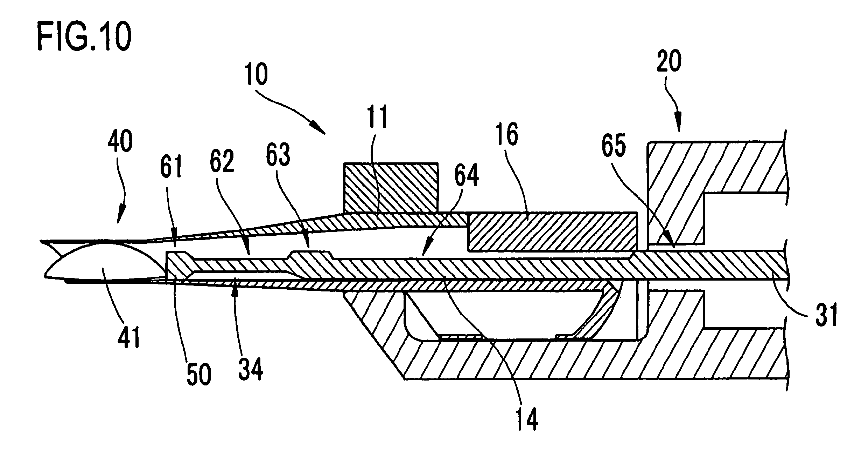

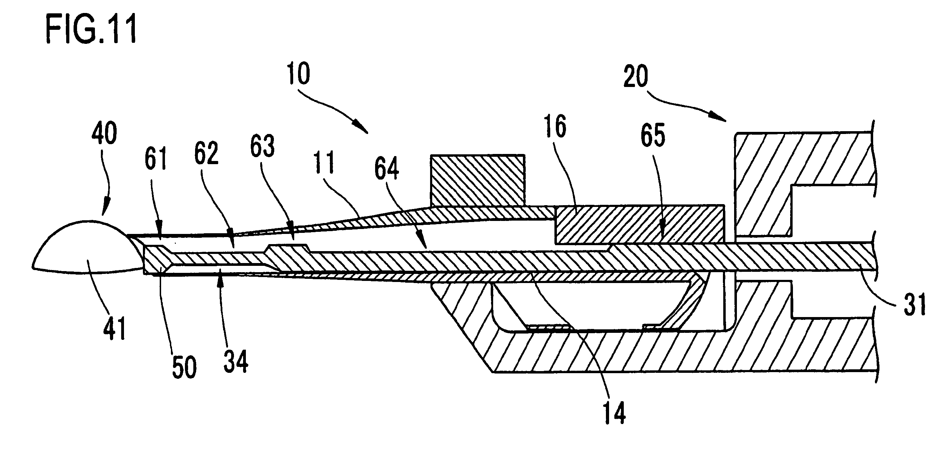

An explanation will be given to changes in operating pressure of the push-out member 30 (the push rod 31) associated with the push-out step of the IOL 40 (a movement step of the push rod 31). Fig. 7 is a schematic sectional view of the cartridge 10 and its surrounding in a state where the head portion 50 is in a contact state with the optic part 41; Fig. 8 is a schematic sectional view of the cartridge 10 and its surrounding in a state where the head portion 50 is released from the contact state with the protrusion 16; Fig. 9 is a schematic sectional view of the cartridge 10 and its surrounding in a state where the optic part 41 starts to be reduced to a smaller diameter by the internal shape of the injection part 11; Fig. 10 is a schematic sectional view of the cartridge 10 and its surrounding in a state where part of the optic part 41 starts to be moved out from the tip end of the injection part 11; and Fig. 11 is a schematic sectional view of the cartridge 10 and its surrounding in a state where the optic part 41 is fully moved out of the injection part 11.

In Figs. 7 and 8, for convenience of explanation, the support part 42 is illustrated on a sectional view and a portion overlapping the head portion 50 and the narrow portion 34 is illustrated by a dotted line. When the IOL 40 is folded inside the cartridge 10, the front (anterior) support part 42 is spread forward by the inner wall of the cartridge 10. On the other hand, the rear (posterior) support part 42 hangs down, without receiving stress, from the rear end (the base end) of the cartridge 10.

As shown in Fig. 7, the contact portion 61 exists at the top of the head portion 50. Accordingly, the head portion 50 will contact with the optic part 41 while the contact portion 61 is in contact with the protrusion 16 (i.e., while the head portion 50 is fitted in the passage formed by the protrusion 16 and the setting stage 14). This prevents upward movement of the push rod 31 and presses the push rod 31 down (against the setting stage 14). The head portion 50 can therefore contact with the optic part 41 without running up on the optic part 41. Furthermore, a frictional force is generated by the contact between the contact portion 61 and the protrusion 16, so that the operating pressure in the push-out step (the operating pressure of the push-out member 30) increases.

When the push rod 31 is axially pushed from the position shown in Fig. 7, the optic part 41 is pushed forward as shown in Fig. 8 to move forward of the protrusion 16. At that time, the contact portion 61 is released from the contact with the protrusion 16. However, at the same time or before the contact portion 61 is released, the next contact portion 63 comes into contact with the protrusion 16 and thus predetermined operating pressure is maintained. In this state, the push rod 31 remains fitted in the passage. Accordingly, the head portion 50 can push the optic part 41 into the injection part 11 without running up on the optic part 41.

Then, the support parts 42 are spread as the optic part 41 is moved forward. Specifically, the front support part 42 is spread along the setting stage 14 and the injection part 11 and the rear support part 42 is spread within the space formed by the narrow portion 34 and the recess 62. Accordingly, the narrow portion 34 and the recess 62 form the space to prevent the rear support part 42 from contacting with the push rod 31 when the rear support part 42 is spread in association with movement of the optic part 41. The length of the recess 62 and the narrow portion 34 (corresponding to an axial length of a region of the push rod 31 that will not contact with the protrusion 16 and the setting stage 14) is determined to be long enough to create a space around the distal end portion of the push rod 31 for receiving the support part 42 to be spread in association with the push-out operation so that the support part 42 can be spread without contacting (interfering) with the moving push rod 31. The length of the recess 62 and the narrow portion 34 has only to be long enough to release the support part 42. If the recess 62 and the narrow portion 34 are too long, the push rod 31 is likely to warp and may cause axis deflection. To avoid such defect, the length of the recess 62 and the narrow portion 34 is preferably determined to be long enough to avoid the support part 42 but as short as possible.

The above configuration can prevent the rear support part 42 from becoming damaged and the head portion 50 from running up on the optic part 41. Thus, the IOL 40 can be pushed out appropriately. To restrain the head portion 50 from running on, the protrusion 16 is preferably positioned above and across the optic part 41.

When the push rod 31 is further pushed forward from the position shown in Fig. 8, the optic part 41 is rolled up to a smaller diameter by the taper tubular shape of the injection part 11 as shown in Fig. 9, so that contact degree (strength) between the IOL 40 (the optic part 41) and the passage inner wall starts to increase. At that time, the contact portion 63 is moved forward beyond the protrusion 16 and released from the contact with the protrusion 16, and thus the recess 64 is positioned under the protrusion 16. On the other hand, even though the frictional force generated between the inner wall of the injection part 11 and the optic part 41 increases, the protrusion 16 does not contact with the push rod 31 and therefore operating pressure of the entire push rod 31 is prevented from changing. In order to offset an increase in frictional force that is generated as the IOL 40 is pushed forward from the setting stage 14 and the optic part 41 is gradually rolled up by the taper tubular shape of the injection part 11, the shape of the push rod 31 or protrusion 16 is determined to gradually reduce contact between the push rod 31 and the protrusion 16. This makes it possible to adjust the operating pressure to an almost constant level for a period from the start of pushing the IOL 40 until just before the IOL 40 is released (until a point at which the operating pressure conventionally suddenly decreases).

As above, it is possible to restrain the change in operating pressure from the start of pushing the IOL 40 until just before it is released. Even if an abnormal condition that the IOL 40 is lodged in the injection part 11 or the like occurs, an operator can easily perceive such abnormal condition by the feeling of operating pressure (e.g., the operating pressure suddenly rises).

When the push rod 31 is further pushed forward from the position shown in Fig. 9, part of the optic part 41 starts to be moved out from the tip end of the injection part 11 and be unfolded as shown in Fig. 10. Herein, in the push-out step from the state of Fig. 9 to the state of Fig. 10, the optic part 41 becomes rolled up inside the injection part 11 and hence the operating pressure in the push-out step is high. Thereafter, when part of the optic part 41 starts to be moved out from the tip end of the injection part 11 and be unfolded, the frictional force between the inner wall of the injection part 11 and the optic part 41 decreases.

When the IOL 40 is further pushed by the push rod 31, the frictional force generated by the close contact between the inner wall (the passage) of the injection part 11 and the optic part 41 according to the degree of movement of the optic part 41 from the tip end of the injection part 11 decreases, thereby greatly changing (decreasing) the operating pressure.

In accordance with the state where part of the optic part 41 starts to be moved out from the tip end of the injection part 11, therefore, the frictional force between the push rod 31 and the protrusion 16 is increased again to prevent the operating pressure from suddenly changing. In this embodiment, in accordance with the state where part of the optic part 41 starts to be moved out from the tip end of the injection part 11, the contact portion 65 comes into contact with the protrusion 16. Accordingly, a predetermined frictional force is generated between the contact portion 65 and the protrusion 16, thus preventing sudden changes in operating pressure.

As the push rod 31 is further moved forward, the entire optic part 41 is pushed out from the tip end of the injection part 11 as shown in Fig. 11. In this state, the friction between the optic part 41 and the injection part 11 disappears and instead the operating pressure in the push-out step is mainly given by a frictional force generated by the contact between the protrusion 16 and the contact portion 65.

As mentioned above, in the push-out step shown in Figs. 7 to 11, the changes in operating pressure in the push-out step can be restrained. It is to be noted that the operating pressure in the push-out step is preferably made almost constant. By changing the frictional force between the push-out member 30 (herein, the push rod 31) and the cartridge 10 (herein, the protrusion 16) in the push-out step, a simple configuration can make the operating pressure almost constant in the IOL push-out step and appropriately push out the IOL. In other words, the operating pressure in the IOL push-out step can be adjusted appropriately. The push rod 31 has only to be formed in a shape (or subjected to a treatment) for discontinuously changing a contact state between the push rod 31 and the protrusion 16 in the axial direction. Specifically, it means such a shape of the push rod 31 as to increase or decrease a frictional force between the push rod 31 and the protrusion 16 according to positions of the IOL in the push-out operation. That is, this shape is not a shape that allows the push rod 31 to continuously contact with the protrusion 16 and receive a constant frictional force.

Operations of the IOL injection instrument 1 having the above configuration are explained below. A surgeon (a user) holds the cartridge 10 while grasping the grip 18 of the cartridge 10 by one hand and picks up the IOL 40 with forceps by the other hand. The user inserts the picked IOL 40 into the cartridge 10 from the rear end thereof so as to put it on the setting stages 14a and 14b. While no stress is applied on the cartridge 10, the half-split elements 12a and 12b are in an open state as shown in Fig. 4A. Accordingly, the IOL 40 set on the setting stages 14a and 14b is retained in an unfolded state (a state where no stress is applied thereon) in the cartridge 10.

To mount the cartridge 10 in the cylindrical unit 20, firstly, the push rod 31 is pulled toward the rear end of the cylindrical unit 20. The user then engages the grip 18 (the rear end) of the cartridge 10 in the recess 23 of the mounting part 21 and pushes down the setting part 12 (the half-split elements 12a and 12b) into the mounting part 21 so as to press the bottom of the mounting part 12 against the protrusions 22 (or the right and left edges of the mounting part 21). When the bottom (the lower part) of the setting part 12 is pressed against the protrusions 22 (or the right and left edges of the mounting part 21), the protrusions 22 will guide the half-split elements 12a and 12b to close into contact with each other. As the setting part 12 is further pushed into the mounting part 21, the half-split elements 12a and 12b are mounted in a closed state in the mounting part 21 as shown in Fig. 4B.

In such a state where the half-split elements 12a and 12b are closed in contact with each other, the width (interval) of the setting stages 14a and 14b is narrow so that the wall surfaces of the setting stages 14a and 14b push the IOL 40 from right and left. The IOL 40 receives stress and thus is folded along the wall surfaces (setting surfaces) of the setting stages 14a and 14b.

After the cartridge 10 is mounted in the mounting part 21, the user inserts the injection part 11 in the patient's eye from which a lens has been removed in advance and then pushes the press part 33 to move the base part 32 and the push rod 31 forward. The contact portion 61 comes into contact with the protrusion 16 and then the head portion 50 comes into contact with the optic part 41. When the press part 33 is further pressed to move the IOL 40 into the injection part 11, the IOL 40 becomes folded (rolled) along the inner wall surface of the injection part 11. At this time, the support parts 42 are spread. The frictional force between the push rod 31 and the protrusion 16 decreases. When the press part 33 is pushed forward, the IOL 40 is gradually pushed out from the tip end of the injection part 11. Accordingly, the contact portion 65 then comes into contact with the protrusion 16 and thus the frictional force between the push rod 31 and the protrusion 16 increases. When the press part 33 is pushed from this state, the entire IOL 40 is pushed (moved) out from the tip end of the injection part 11.

In a sequence of operations in the above push-out step of the IOL 40, the operating pressure to the push-out member 30 is restrained from changing (preferably, the operating pressure is made almost constant). Specifically, the operating pressure is prevented from changing in all stages including a stage before the head portion 50 comes into contact with the IOL 40 (i.e., the head portion 50 contacts with the protrusion 16), a stage in which the folded IOL 40 is further reduced to a smaller diameter by the internal shape of the injection part 11, and a stage in which part of the IOL 40 starts to move out from the tip end of the injection part 11 (i.e., the frictional force between the IOL 40 and the injection part 11 starts to decrease). This makes it easy to handle the IOL injection instrument 1 in the push-out step of the IOL 40. Furthermore, the operating pressure during normal operation in the push-out step of the IOL 40 is prevented from changing and therefore it is easy for the user to perceive that the IOL 40 is lodged somewhere or another abnormal condition and recognize whether or not the push-out member 30 is normally operated.

In the aforementioned embodiment, the push rod 31 is designed so that its outer surface changes in shape in the axial direction to change a contact degree between the push rod 31 and the protrusion 16 provided on the inner wall of the passage in the cartridge 10, thereby producing different frictional forces between the protrusion 16 and the push rod 31 to adjust the operating pressure of the push-out member 30 to an almost constant level. However, the present invention is not limited to such configuration. Any other configurations may be adopted if only an interfering part is provided in the passage of the push rod to produce different frictional forces between the push-out member 30 and the interfering part in the push-out step so that the operating pressure of the push-out member 30 (the push rod 31) is adjusted to an almost constant level. For instance, the push rod 31 may be made from a thin rod axially extending with a uniform diameter so that this push rod 31 always contacts with the protrusion 16 in the push-out step. As another alternative, the push rod may be subjected to surface treatment to polish or roughen the surface of a certain region along the axial direction of the push rod to produce different frictional forces between contact surfaces of the protrusion 16 and the push rod 31 in the push-out step.

In the aforementioned embodiment, the push rod 31 and the protrusion 16 provided in the cartridge 10 contact with each other, producing a frictional force. The present invention is not limited thereto and may be applied to any other configuration if only making the operating pressure of the advancing push-out member 30 almost constant. For instance, a configuration may be adopted in which an interfering part is provided in a (tube) inner wall of the passage of the push rod to bring the base part 32 (included in the push rod of the push-out member) into contact with the cartridge 10 or a member provided in the cylindrical unit 20, thereby generating a frictional force. Concretely, for example, a recess is formed in the outer surface of the base part 32 and a protrusion serving as the interfering part is formed on the inner wall of the cylindrical unit 20.
It is to be noted that the inner wall of the cartridge 10 is included in the tube inner wall.

In the aforementioned embodiment, the push rod 31 comes into contact with the protrusion 16 when the IOL 40 starts to be moved out from the injection part 11, thereby to prevent the operating pressure of the push-out member 30 from changing during movement of the IOL 40. The present invention may be applied to any other configuration that generates a frictional force between the push-out member 30, the cartridge 10, and the cylindrical unit 20 to prevent the operating pressure of the push-out member 30 from changing during movement of the IOL 40 from the tip end of the injection part 11. For instance, the head portion 50 may be formed with a pair of protrusions horizontally protruding from the outer surface of the head portion 50 (the protrusions are formed on both side surfaces of the head portion 50). These pair protrusions will contact with the inner wall of the injection part 11 as the head portion 50 advances. Thus, the pair protrusions serve to increase the frictional force between the push rod 31 and the injection part 11 when the IOL 40 starts to be moved out from the tip end of the injection part 11 and hence the frictional force starts to decrease between the IOL 40 and the injection part 11. This makes it possible to prevent the operating pressure of the push-out member 30 from changing when the IOL 40 is moved out of the injection part 11. The pair protrusions are not limited to the configuration that horizontally protrudes from the head portion 50. Any other configuration may be adopted if only a pair of protrusions protrudes from the head portion 50 to contact with the inner wall of the injection part 11. For instance, the protrusions may be provided extending vertically. Alternatively, the protrusions may be provided in the push rod 31.

In the aforementioned embodiment, the push rod 31 has an outer shape that contacts with the protrusion 16 extending above and along the axial direction of the push rod 31 or a shape that does not contact with the protrusion 16. Furthermore, another shape may be added to provide different contact degrees (strength) between the interfering part and the push rod to decrease a frictional force therebetween. For instance, the outer surface of the push rod 31 which contacts with the protrusion 16 may be designed to have different widths (in a direction perpendicular to a moving direction of the push rod 31) to produce stepwise different frictional forces between the protrusion 16 and the push rod 31. Alternatively, the intervals between the protrusion 16 and the setting stage 14 which form the passage allowing the push rod to move through may be different in the axial direction. The above configurations can finely adjust the operating pressure of the push-out member 30.

In the aforementioned embodiment, the IOL 40 having been folded in advance on the setting stage 14 is pushed out by the push-out member 30. However, the present invention is not limited thereto and may be applied to another configuration if only the IOL 40 is pushed out in a folded (rolled) state from the injection part 11. For instance, it may be arranged such that the IOL placed in a state where no stress is applied thereto on the setting stage in the cartridge (the lens holding part) is folded along the internal shape of the injection part or the like in the push-out step and moved out from the tip end of the injection part.

In the aforementioned embodiment, near behind the head portion 50, the narrow portion 34 and the recess 62 are formed with a smaller diameter than the diameter of the push rod 31. This shape is to avoid the open-loop-shaped support parts 42 of the IOL 40 and is not always required. In the case of using an IOL having support parts each of which is not formed in an open-loop shape, the support parts are unlikely to be damaged in the push-out step and therefore the narrow portion 34 and the recess 62 may not be provided.

While the presently preferred embodiment of the present invention has been shown and described, it is to be understood that this disclosure is for the purpose of illustration and that various changes and modifications may be made without departing from the scope of the invention as set forth in the appended claims.

### Reference Signs List

- 1: IOL injection instrument
- 10: Cartridge
- 20: Cylindrical unit
- 30: Push-out member
- 31: Push rod
- 34: Narrow portion
- 40: IOL
- 42: Support part
- 50: head portion
- 61, 63, 65: Contact portion
- 62, 64: Recess

## Claims

1. An intraocular lens injection instrument comprising:
a lens holding part (10) comprising:
a setting part (12) in which an intraocular lens (IOL) (40) is placeable; and
an injection part (11) having a taper tubular shape for rolling up the IOL (40) placed in the setting part (12);
a main body (20) having a tubular shape and integrally or removably holding the lens holding part (10) in a front end; and
a push-out member (30) including a push rod (31) provided to be movable forward in an axial direction thereof in the main body (20) and the lens holding part (10) to push the IOL out of a tip end of the injection part (11),
**characterized in that**
a member provided in the main body (20) or the lens holding part (10) is internally formed with an interfering part (16) that will contact with the push-out member (30) to generate a frictional force to adjust operating pressure of the push-out member (30) during forward movement of the push-out member (30), and
the push-out member (30) is applied with treatment or a shape for discontinuously changing a contact state with the interfering part (16) in the axial direction to change the frictional force with respect to the interfering part (16) according to a position of the IOL with respect to the lens holding part (10).

2. The IOL injection instrument according to claim 1 wherein
the interfering part (16) is formed on an inner wall of a movement passage of the push rod (31) in the member provided in the body or the lens holding part (10).

3. The IOL injection instrument according to claim 2, wherein
the interfering part (16) is a protrusion (16) provided at an upper portion of the inner wall of the movement passage of the push rod (31) in the member provided in the lens holding part (10).

4. The IOL injection instrument according to one of claims 1 to 3, wherein
the interfering part (16) is a protrusion (16) provided on an upper portion of an inner wall of the setting part (12), the protrusion (16) guiding a folding direction of the IOL (40) along the inner wall surface of the setting part (12) when the IOL (40) placed in the setting part (12) is to be folded.

5. The IOL injection instrument according to one of claims 1 to 4, wherein the push-out member (30) is provided with a contact portion (61, 63, 65) that will contact with the interfering part (16) and a recess (62, 64) that will not contact with the interfering part (16), the contact portion (61, 63, 65) and the recess (62, 64) being discontinuously arranged in the axial direction.

6. The IOL injection instrument according to one of claims 1 to 5, wherein
the interfering part (16) is provided on an inner wall of the injection part, and
the push-out member (30) is applied with the treatment or the shape for changing the contact state with the interfering part (16) in the axial direction to change the frictional force with respect to the interfering part (16) according to the position of the IOL (40) in the injection part (11) so as to make the operating pressure of the push-out member (30) almost constant while the IOL (40) is moved forward and rolled in the injection part (11).

## Patentansprüche

1. Intraokularlinsen-Injektionsinstrument, das aufweist:
einen Linsenhalteabschnitt (10), der aufweist:
einen Einsetzabschnitt (12), in den eine intraokulare Linse (IOL) (40) einbringbar ist; und
einen Injektionsabschnitt (11), der eine verjüngte Röhrenform hat, zum Aufrollen der IOL (40), die in dem Einsetzabschnitt (12) platziert ist;
einen Hauptkörper (20), der eine Röhrenform hat und integral oder entfernbar den Linsenhalteabschnitt (10) in einem vorderen Ende hält; und
ein Ausstoßbauteil (30), das einen Druckstab (31) enthält, der so vorgesehen ist, dass er vorwärts in einer axialen Richtung in dem Hauptkörper (20) und dem Linsenhalteabschnitt (10) bewegbar ist, um die IOL aus einem spitzen Ende des Injektionsabschnitts (11) herauszudrücken,
**dadurch gekennzeichnet, dass**
ein Bauteil, das in dem Hauptkörper (20) oder dem Linsenhalteabschnitt (10) innen mit einem Eingriffsabschnitt (16) ausgebildet ist, der mit dem Ausstoßbauteil (30) in Kontakt kommt, um eine Reibungskraft zu erzeugen, um den Betriebsdruck des Ausstoßbauteils (30) während der Vorwärtsbewegung des Ausstoßbauteils (30) anzupassen, und
das Ausstoßbauteil (30) mit einer Behandlung oder einer Form zum unstetigen Verändern eines Kontaktzustands mit dem Eingriffsabschnitt (16) in der axialen Richtung eingesetzt ist, um die Reibungskraft bezüglich dem Eingriffsabschnitt (16) gemäß einer Position der IOL mit Bezug zu dem Linsenhalteabschnitt (10) zu ändern.

2. IOL-Injektionsinstrument gemäß Anspruch 1, wobei
der Eingriffsabschnitt (16) an einer inneren Wand einer Bewegungspassage des Druckstabs (31) in dem Bauteil, das in dem Körper oder dem Linsenhalteabschnitt (10) vorgesehen ist, ausgebildet ist.

3. IOL-Injektionsinstrument gemäß Anspruch 2, wobei
der Eingriffsabschnitt (16) ein Vorsprung (16) ist, der an einem oberen Abschnitt der inneren Wand der Bewegungspassage des Druckstabs (31) in dem Bauteil, das in dem Linsenhalteabschnitt (10) vorgesehen ist, vorgesehen ist.

4. IOL-Injektionsinstrument gemäß einem der Ansprüche 1 bis 3, wobei
der Eingriffsabschnitt (16) ein Vorsprung (16) ist, der an einem oberen Abschnitt einer inneren Wand des Einsetzabschnitts (12) vorgesehen ist, wobei der Vorsprung (16) eine Faltrichtung der IOL (40) entlang der inneren Wandfläche des Einsetzabschnitts (12) leitet, wenn die IOL (40) in dem Einsetzabschnitt (12) zu falten ist.

5. IOL-Injektionsinstrument gemäß einem der Ansprüche 1 bis 4, wobei
das Ausstoßbauteil (30) mit einem Kontaktabschnitt (61, 63, 65) vorgesehen ist, der mit dem Eingriffsabschnitt (16) in Kontakt kommt, und mit einer Aussparung (62, 64), die nicht mit dem Eingriffsabschnitt (16) in Kontakt kommt, wobei der Kontaktabschnitt (61, 63, 65) und die Aussparung (62, 64) diskontinuierlich in der axialen Richtung angeordnet sind.

6. IOL-Injektionsinstrument gemäß einem der Ansprüche 1 bis 5, wobei
der Eingriffsabschnitt (16) an einer inneren Wand des Injektionsabschnitts vorgesehen ist, und
das Ausstoßbauteil (30) mit der Behandlung oder der Form zum Verändern des Kontaktzustands mit dem Eingriffsabschnitt (16) in der axialen Richtung eingesetzt ist, um die Reibungskraft bezüglich dem Eingriffsabschnitt (16) gemäß der Position der IOL (40) in dem Injektionsabschnitt (11) zu verändern, um den Betriebsdruck des Ausstoßbauteils (30) nahezu konstant zu halten, während die IOL (40) vorwärts bewegt wird und in dem Injektionsabschnitt (11) zusammengerollt wird.

## Revendications

1. Instrument d'injection de lentille intraoculaire comprenant :
une partie de support de lentille (10) comprenant :
une partie de placement (12) dans laquelle une lentille intraoculaire (IOL) (40) peut être placée ; et
une partie d'injection (11) présentant une forme tubulaire conique pour enrouler l'IOL (40) placée dans la partie de placement (12) ;
un corps principal (20) présentant une forme tubulaire et contenant de manière intégrale ou de manière amovible la partie de support de lentille (10) dans une extrémité avant ; et
un élément de sortie (30) comprenant une tige de poussée (31) prévue de manière à pouvoir être déplacée en avant dans une direction axiale de celle-ci dans le corps principal (20) et la partie de support de lentille (10) pour pousser l'IOL hors d'une extrémité terminale de la partie d'injection (11),
**caractérisé en ce que**
un élément prévu dans le corps principal (20) ou dans la partie de support de lentille (10) comporte intérieurement une partie interférente (16) qui viendra en contact avec l'élément de sortie (30) pour générer une force de frottement pour ajuster la pression d'actionnement de l'élément de sortie (30) pendant un mouvement en avant de l'élément de sortie (30), et
l'élément de sortie (30) reçoit un traitement ou une forme pour modifier de manière discontinue un état de contact avec la partie interférente (16) dans la direction axiale pour modifier la force de frottement par rapport à la partie interférente (16) en fonction d'une position de l'IOL par rapport à la partie de support de lentille (10).

2. Instrument d'injection d'IOL selon la revendication 1, dans lequel
la partie interférente (16) est formée sur une paroi interne d'un passage de mouvement de la tige de poussée (31) dans l'élément prévu dans le corps ou dans la partie de support de lentille (10).

3. Instrument d'injection d'IOL selon la revendication 2, dans lequel
la partie interférente (16) consiste en une protubérance (16) prévue au niveau d'une partie supérieure de la paroi interne du passage de mouvement de la tige de poussée (31) dans l'élément prévu dans la partie de support de lentille (10).

4. Instrument d'injection d'IOL selon l'une des revendications 1 à 3, dans lequel
la partie interférente (16) consiste en une protubérance (16) prévue sur une partie supérieure d'une paroi interne de la partie de placement (12), la protubérance (16) guidant une direction de pliage de l'IOL (40) le long de la surface de paroi interne de la partie de placement (12) lorsque l'IOL (40) placée dans la partie de placement (12) doit être pliée.

5. Instrument d'injection d'IOL selon l'une des revendications 1 à 4, dans lequel l'élément de sortie (30) est pourvu d'une partie de contact (61, 63, 65) qui viendra en contact avec la partie interférente (16) et d'un évidement (62, 64) qui ne viendra pas en contact avec la partie interférente (16), la partie de contact (61, 63, 65) et l'évidement (62, 64) étant agencés de manière discontinue dans la direction axiale.

6. Instrument d'injection d'IOL selon l'une des revendications 1 à 5, dans lequel
la partie interférente (16) est prévue sur une paroi interne de la partie d'injection, et
l'élément de sortie (30) reçoit le traitement ou la forme pour modifier l'état de contact avec la partie interférente (16) dans la direction axiale pour modifier la force de frottement par rapport à la partie interférente (16) en fonction de la position de l'IOL (40) dans la partie d'injection (11) de manière à rendre la pression d'actionnement de l'élément de sortie (30) presque constante alors que l'IOL (40) est déplacée en avant et enroulée dans la partie d'injection (11).
